# EUROPEAN PATENT APPLICATION

(11) **EP 1 975 614 A1**
(43) Date of publication of application: **01.10.2008**
(21) Application number: 06842977.8
(22) Date of filing: 21.12.2006
(51) Int. Cl.: G01N 33/53, C07K 14/705

(54) **METHOD FOR PREDICTION OF PROGNOSIS OF ACUTE CORONARY SYNDROME**

(30) Priority: 22.12.2005 JP 2005370030
(71) Applicant: Shionogi Co., Ltd., Osaka-shi, Osaka 541-0045 (JP)
(72) Inventor: KUME, Noriaki, Kyoto 606-8501 (JP); KOMINAMI, Goro, Osaka 553-0002 (JP)
(74) Representative: Spencer, Matthew Peter
(86) International application number: PCT/JP2006/325469
(87) International publication number: WO 2007/072896

(57) **Abstract**

Problem is to provide a diagnostic marker for prognostic prediction of acute coronary syndrome. Means to solve the Problem is to make it possible to predict the risk of recurrence of acute coronary syndrome by measuring the concentration of soluble LOX-1 in the blood.

## Description

### [Technical Field]

This invention pertains to a method of predicting the risk of future recurrence of acute coronary syndrome and to a kit for the implementation of said method.

### [Background Art]

The series of conditions from unstable angina to acute myocardial infarction, and further to sudden cardiac death complicated by these, are inclusively referred to as acute coronary syndrome (ACS). These are all considered to be conditions in which narrowing or blockage of the coronary arteries occurs as the result of the rupture of an atheroma (plaque), which develops through arteriosclerosis of the coronary arteries that supply nutrients to the heart. While acute coronary syndrome is currently on the rise in modern society, since the majority of cases occur suddenly and without warning, they often cannot be resuscitated and result in sudden death. Even if the patient were quickly admitted to a hospital, emergency heart surgery or emergency percutaneous transluminal coronary angioplasty (percutaneous coronary intervention) (PCI), etc. is often required to save the patient's life, and the medical economic burden cannot be computed.

The recurrence of acute coronary syndrome can now be prevented to some extent through treatment with antithrombotics and cholesterol-lowering drugs (statins) and the like, but even so, a large-scale clinical trials has shown that statins provide a decrease in recurrence in approximately 30% of patients, while recurrence cannot be prevented in the remaining 70% of patients by treatment with these kinds of drugs. Consequently, while new treatment protocols are anticipated to completely prevent the recurrence of acute coronary syndrome, no such therapies currently exist. Under these circumstances, it is believed that the accurate identification of high-risk cases and prevention of attacks through focused management, or barring this, the prevention of sudden outpatient attacks, will be useful in saving lives and improving prognoses.

Currently, prognoses for recurrence are made in clinics by mechanical examinations, such as heart catheter examinations, including coronary angiography or left ventriculography, or using biomarkers. However, the former are not easily implemented from time and cost aspects, and place a decidedly heavy load on the patient. In addition, they do not provide complete information from the viewpoint of predicting recurrence. For example, research until now has shown that the risk of recurrence cannot be predicted by the degree of restriction in the coronary artery in coronary angiography (see non-patent reference 4, below) In the latter, acute stage markers, such as troponin T, H-FABP, and high-sensitivity CRP, etc., or risk factor markers, such as total cholesterol, triglycerides, HDL cholesterol, and remnant lipoprotein (RLP) cholesterol, etc., are used, but they are not sufficiently effective as markers for predicting recurrence for reasons of sensitivity and specificity(see non-patent reference 5, below).

It has become clear in recent years that acute coronary syndrome episodes are attributed to the formation of occlusive thrombus produced by the rupture or continuous erosion of arterial atherosclerotic plaque. The inflammatory response and oxidation stress in the vessel walls play crucial roles in plaque rupture and erosion, but of these, functional disorders of the vessel wall, primarily increased protease activity and apoptosis (cell death), initiated by oxide-denatured LDLs (low-density lipoproteins), are known to be the primary causes. LOX-1 (Lectin-like oxidized low-density lipoprotein receptor-1) has been identified as a receptor protein for these oxidized LDLs (see non-patent reference 1, below). In the body, this LOX-1 is expressed on cell surfaces as a common membrane protein, but it is known that protease action will cleave this in the extracellular domain of the transmembrane region as soluble LOX-1 (see non-patent reference 2, below). It is also reported that this soluble LOX-1 may be the primary diagnostic marker for acute coronary syndrome because its blood concentrations increase markedly in the acute phase of acute coronary syndrome (see non-patent reference 3, below).However, its potency has been unknown.
[Non-patent Reference 1] Nature, 1997, Vol. 386, pp. 73-77
[Non-patent Reference 2] Arterioscler. Thromb. Vasc. Biol., 2000, 20(3), pp. 715-720
[Non-patent Reference 3] Circulation, 2005, 112(6), pp. 812-818
[Non-patent Reference 4] Levine GN, et al.: N. Engl. J. Med., 1995, Vol. 332, pp. 512-521: Libby, Circulation, 1995, Vol. 91, pp. 2844-2850
[Non-patent Reference 5] M.Panteghini, Role and importance of biochemical markers in clinical cardiology: European Heart J., 2004, Vol.25, pp1187-1196

### [Disclosure of Invention]

### [Problem to be solved by the Invention]

The purpose of this invention is to provide a high sensitivity prognostic prediction method to prevent the recurrence of acute coronary syndrome.

### [Means of Solving Problem]

The inventors tracked the prognoses for patients who had suffered acute coronary syndrome for approximately 7 years to study the relationship between blood concentrations of soluble LOX-1 and the rate of recurrence of acute coronary syndrome. Then, as the result of discovering a high correlation between soluble LOX-1 concentrations and the rate of acute coronary syndrome recurrence, the inventors arrived at this invention.

### [Advantages of the Invention]

It is possible to predict the recurrence of acute coronary syndrome by measuring the soluble LOX-1 concentration in a test specimen taken from the test subject according to this invention.

### [Brief Description of Drawings]

[Fig. 1] This is a standard curve for human soluble LOX-1.
[Fig. 2] This is a graph showing the median, and 10^{th}, 25^{th}, 75^{th}, and 90^{th} percentiles in the results of measuring the human soluble LOX-1 in a group of patients without recurrence of acute coronary syndrome and a group of patients with recurrence of acute coronary syndrome or death.
[Fig. 3] This is a graph showing the median, and 10^{th}, 25^{th} 75^{th}, and 90^{th} percentiles in the results of measuring the high-sensitivity CRP in a group of patients without recurrence of acute coronary syndrome and a group of patients with recurrence of acute coronary syndrome or death.
[Fig. 4] This is a graph showing the median, and 10^{th}, 25^{th} , 75^{th} , and 90^{th} percentiles in the results of measuring the left ventricular ejection fraction (LVEF) by echocardiography in a group of patients without recurrence of acute coronary syndrome and a group of patients with recurrence of acute coronary syndrome or death.

[Fig. 5] This is a graph showing the median, and 10^{th}, 25^{th} 75^{th}, and 90^{th} percentiles in the results of measuring the troponin T in a group of patients without recurrence of acute coronary syndrome and a group of patients with recurrence of acute coronary syndrome or death.
[Fig. 6] This uses a Kaplan-Meir curve to show the results of tracking the recurrence of acute coronary syndrome (including death) when the cutoff for blood concentration of human soluble LOX-1 was set at 5ng/mL.
[Fig. 7] This uses a Kaplan-Meir curve to show the results of tracking the recurrence of acute coronary syndrome (including death) when the cutoff for blood concentration of human soluble LOX-1 was set at 3ng/mL.

### [Preferred Embodiment of the Invention]

This invention pertains to a method for screening test subjects with high risk for recurrence of acute coronary syndrome, which includes a process to measure the concentration of soluble LOX-1 in a test specimen taken from the test subject, wherein a condition in which the concentration in the test specimen is greater than a reference value is taken as an indicator that the aforementioned test subject is at high risk for recurrence of acute coronary syndrome. If this method is used, test subjects with high risk for recurrence of acute coronary syndrome can be easily screened, and the risk of said occurrence can be predicted.

Concretely, this invention includes a process to measure the concentration of soluble LOX-1 in a test specimen taken from the test subject. "Test subject" is not specifically limited, but it is preferable that they are a person who is susceptible to acute coronary syndrome (including people who have been deemed to have suffered from it as the result of measurement) or one who is suspected might be susceptible to acute coronary syndrome.

A specimen from the aforementioned test subject (hereinafter, "test specimen") is collected. Test specimens include those derived from urine, whole blood, plasma, serum, or blood, but either plasma or serum is preferred.

Next, the soluble LOX-1 concentration in the aforementioned test specimen is measured. The soluble LOX-1 in this invention is produced by, e.g., using a protease to cleave a portion of the extracellular domain of human LOX-1 shown in the amino acid sequence in Sequence No. 1. A variety of soluble LOX-1 is present in the blood, which differ depending on the cleavage site. Typical examples of such amino acid sequences include those listed, e.g., in Sequence Nos. 2 and 3, but one to five amino acids may be deleted, added, or substituted to these. At this point, it is possible to measure the soluble LOX-1 concentration by a variety of methods known to a person skilled in the art (e.g., western blotting, radio immunoassay), but ELISA, which uses antibodies or antibody fragments that specifically bind to human soluble LOX-1, generally is preferred. Antibodies specifically recognize the soluble LOX-1 polypeptide or a portion thereof. Such antibodies can be obtained by the usual methods (e.g., Shin Seikagaku Shiken Kouza 1, Protein 1, p. 389, 1992). Additionally, monoclonal antibodies can also be obtained by commonly known methods (Tankuron Koutai, Kodansha Scientific, 1983). It is then possible to use these antibodies as the constituents of a diagnostic kit for measuring soluble LOX-1 concentrations in test specimens, or as diagnostic markers.

To describe it in detail, the principle of ELISA is based on the enzyme immunoassay method. Namely, with this method, e.g., first, an anti-human soluble LOX-1 antibody, preferably a monoclonal antibody (first antibody), is solid phased onto a solid support (e.g., plate), and then blocked with a non-specific protein (e.g., bovine serum albumin) to prevent nonspecific adsorption. Next, human soluble LOX-1 standard solution or test specimen is added to the aforementioned solid phased plate and reacted. After the reaction, the plate is rinsed, and an enzyme marker-labeled anti-human soluble LOX-1 antibody (second antibody) is added and reacted. After rinsing the plate, a matrix is added to perform an enzyme reaction and the enzyme activity remaining on the plate is read as light absorbance.

In the above, a polyclonal antibody may be used as the first antibody and a monoclonal antibody may be used as the second antibody. According to the aforementioned method, the higher the soluble LOX-1 concentration in the standard solution or test specimen used, the stronger the enzyme activity (absorbance) is deemed. The concentration of soluble LOX-1 contained in a test specimen can be easily read by plotting the absorbance of the aforementioned standard solution to produce a standard curve (calibration curve), and then comparing the absorbance of the test specimen with said standard curve. The risk of developing acute coronary syndrome for the test subject can be judged by comparing this value with a preset reference value (cutoff).

Concretely, in the event that the resulting measured value is compared with the reference value and is higher than the reference value, the test subject is identified as in the group with high risk of recurrence of acute coronary syndrome, i.e., is screened as the high-risk group. The reference value is dependent on the age, gender, other morbid diseases, etc. of the test subject, but a person skilled in the art can set other reference values corresponding to false-positive and false-negative results that can be permitted for each individual patient. For example, when serum is drawn from a test subject in the acute phase of acute coronary syndrome and used as the test sample, 3 ng/mL can be set as that reference value. A test subject who exceeds that reference value is said to be at very high risk for recurrence of acute coronary syndrome. Additionally, the measured values of a control group of random people who have never suffered from acute coronary syndrome in the past, or the value previously measured for the same patient, may also be utilized as the reference value to predict risk.

As above-mentioned, this invention can provide extremely useful information to predict recurrence of acute coronary syndrome. This invention pertains to a method for screening test subjects with high risk for recurrence of acute coronary syndrome, which includes a process to measure the concentration of soluble LOX-1.Additionaly, this invention pertains to use of soluble LOX-1 as a marker for predicting the recurrence of acute coronary syndrome. Further, this invention pertains to a kit, which can be implemented in the aforementioned method. This kit includes specific antibody against human soluble LOX-1 and thereof fragments. The kit of this invention and the method of measuring soluble LOX-1 concentration in test specimens utilizing said kit will be described in detail below.

It is preferable to use serum or plasma as the test specimens in the measurement method employing the kit of this invention, and these can be prepared from withdrawn blood according to the usual method.
A monoclonal antibody or polyclonal antibody against human soluble LOX-1 (anti-human soluble LOX-1 antibody) is the essential constituent of the kit of this invention, and it is preferred that this be respectively combined with a polyclonal antibody or monoclonal antibody against soluble LOX-1, or combined with two types of monoclonal antibodies.
It is preferred that the antibody solidified onto the plate is a monoclonal antibody, and it may be used in pre-solidified plate form. It may also be used in its original affinity gel form, or it is possible to prepare it in a suitable container or test tube that can be shaken or centrifuged to remove the unbound fraction of the affinity gel.

It is also preferred that the aforementioned solid phased plate or gel is buffered in advance with a suitable buffer solution. Furthermore, the aforementioned solid phased plate or gel may also contain a common preservative, such as ProClin 150, etc. It is further preferred that enzyme-labeled anti-human soluble LOX-1 antibody is supplied as the second antibody for ELISA as a constituent of the kit of this invention. Further, stabilizers and/or preservatives, such as bovine serum albumin, etc., may also be admixed into the kit of this invention as necessary. This preservative shall be selected from those that will not affect the test values when used in the kit.

The kit of this invention may also contain a surfactant, such as 3-[(3-cholamidopropyl) dimethylammonio] propane sulfonic acid, etc., or a water-soluble or water-miscible glycerin, alcohol, or glycol, to prevent adsorption, and may contain a mixed organic solvent, etc., such as ethanol and diethylether or chloroform and methanol, as a defatting agent.

### [Working Examples]

### Working Example 1

### (1) Preparation of polyclonal antibody to label soluble LOX-1

E. coli expressing human LOX-1 extracellular domain (his-tagged) were cultured, and human LOX-1 extracellular domain protein was refined from this E. coli using a Ni-NTA gel column. Physiological saline solution containing approximately 0.8mg/mL of this protein was thoroughly mixed with an equal quantity of Freund's complete adjuvant to form an emulsion. 0.5 mL of this emulsion was then inoculated 6 times at 3-week intervals into the backs of rabbits (Japanese white, female). The rabbits were exsanguinated on the 10^{th} day after the final inoculation was completed to obtain antiserum.

### (2) Preparation of enzyme-labeled antibodies

4.61 mg enzyme (horse radish peroxidase) was dissolved into 0.4 mL of 0.1 mol/L phosphate buffer solution (pH 6.0), to which 0.4 mg succinimidyl 4-(N-maleimidomethyl) cyclohexane-1-carboxylate was added and caused to react for 60 minutes at 30°C. The reaction solution was refined using a gel filtration column to obtain 3.10 mg of maleimide-activated enzyme.

Meanwhile, after pepsin digesting its IgG fraction, the antiserum was refined with a gel filtration column and obtained a F(ab')₂ fragment. Then, reduced the F(ab')₂ fragment with mercaptoethylamine, refined it with a gel filtration HPLC, and obtained the Fab' fragment.
After reacting the 3.10 mg of obtained maleimide-activated enzyme and 3.11 mg of the Fab' fragment in 0.52 mL of 0.1 mol/1 phosphate buffer solution (pH 6.0) for 18 hours at 4°C, refined with gel filtration HPLC and obtained 3.47 mg enzyme-labeled antibody. An average of 1.0 enzyme molecules were introduced per one molecule of the Fab' fragment of the resulting enzyme-labeled antibody. After adding ProClin 150 to 0.1vol% to the enzyme-labeled antibody solution as a preservative, the solution was divided and stored frozen at -80°C.

### (3) Preparation of antibody solid phased plates

After admixing 0.1 mg of IgG fraction of antiserum to 10mL of 0.1 mol/L phosphate buffer solution containing 0.1 vol% ProClin 150 (pH 7.0), 0.1 mL was added to each well in a 96-well microplate and left still overnight at room temperature. This was rinsed twice with approximately 0.2 mL measurement buffer solution (0.5 g/dL bovine serum albumin, 0.1g/dL 3-[(3-cholamidepropyl) dimethylammonio] propane sulfonic acid, and 0.1 mol/L phosphate buffer solution containing 0.1 vol% ProClin 150, pH 7.0), and then 0.2 mL measurement buffer solution was added and blocking was performed for over 2 hours at room temperature.

### (4) ELISA

After adding 0.1 mL measurement buffer solution and 0.01 mL standard sample solution or human serum specimen to a well in the antibody solid phased plate and incubating for 2 hours at room temperature, the well was rinsed twice with approximately 0.2 mL measurement buffer solution, Next, 0.1 mL enzyme-labeled antibody solution diluent (approximately 900 ng/mL) was added and incubated for 16 hours at room temperature, then rinsed twice with approximately 0.2 mL measurement buffer solution, after which, 0.1 mL matrix solution (e.g., TMB+, made by DAKO) was added and left still for precisely 30 minutes at room temperature and shielded from light. The reaction was stopped with 0.1 mL of 0.5 mol/L sulfuric acid, and the 450nm light absorbance was measured with a plate reader.

### (5) ELISA standard curve

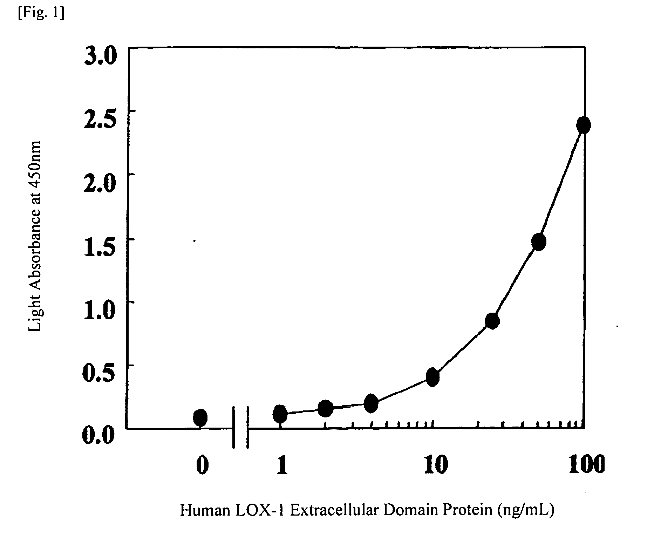

An ELISA method capable of measuring soluble LOX-1 in human serum was established with a 1 ng/mL quantitation limit and 1-100 ng/mL quantitation range. A sample standard curve is shown in Fig. 1.

### Working Example 2

### Utility of soluble LOX-1 in prognosis of recurrence after acute coronary syndrome episode

The aforementioned ELISA method was used to measure the soluble LOX-1 concentrations contained in peripheral venous blood collected from acute coronary syndrome patients (107 cases) during the acute phase in the initial onset of acute coronary syndrome. The subsequent clinical courses of all these patients were then tracked for approximately 7 years, at most, and based on those results were divided into a group of patients with recurrent acute coronary syndrome (including patients who died as a result) (15 cases) and a group of patients for whom it did not recur (88 cases). And, Kruskal-Wallis testing (Shinban Igakuheno toukeigaku, Asakura Syoten, 1993) was applied to the correlative relationship of soluble LOX-1 concentration in each group.
Furthermore, the blood troponin T concentrations (89 cases), blood high-sensitivity CRP levels (71 cases) and left ventricular ejection fraction (LVEF) (88 cases), which were used as primary clinical marker of acute coronary syndrome ,were compared with soluble LOX-1. Then, troponin T was measured by an electrochemical light emission immunoassay kit (Roche), high-sensitivity CRP was measured by an immunonephelometry assay kit (Dade Behring), and LVEF was measured by echocardiography. Manufacturer's instructions were followed for the specific measurement techniques.

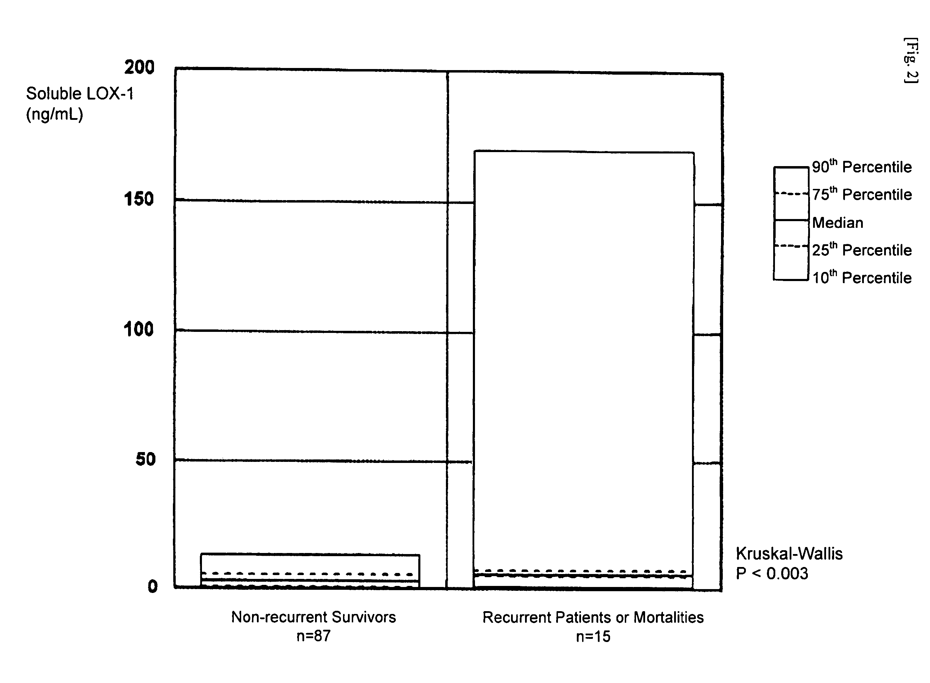

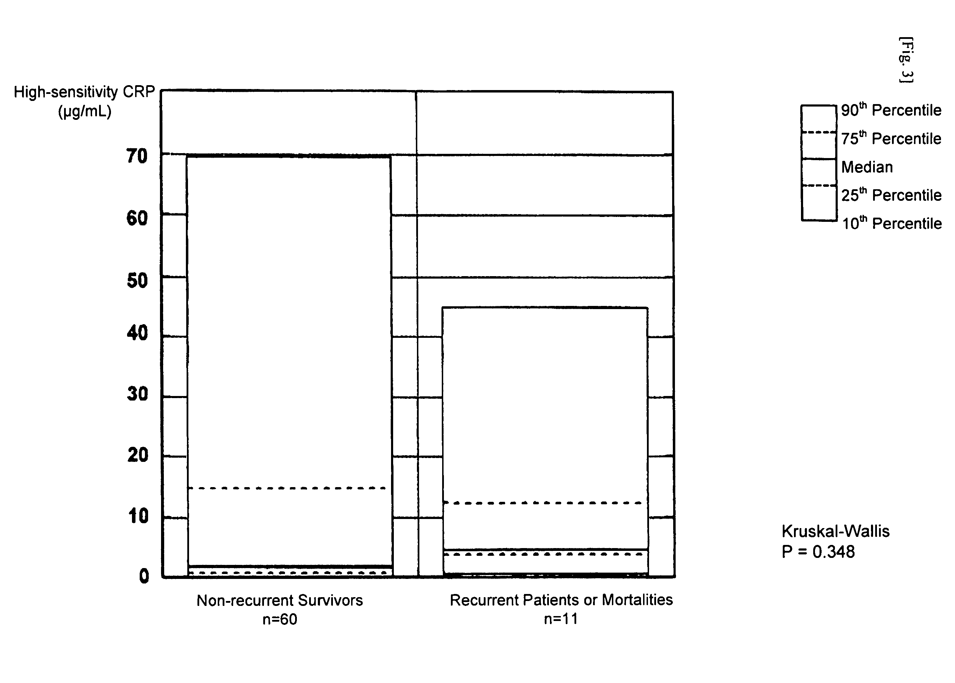

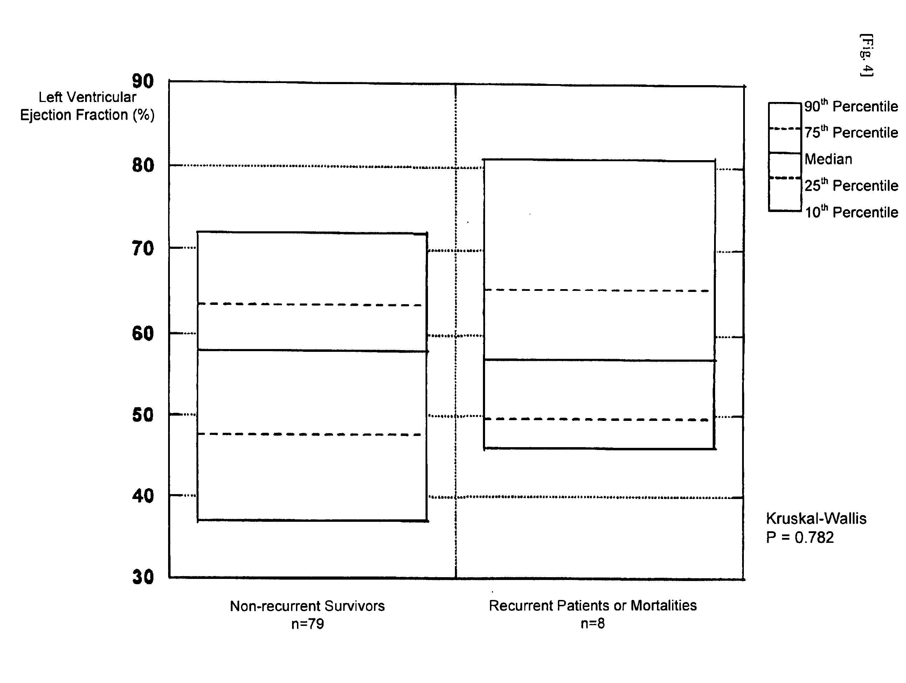

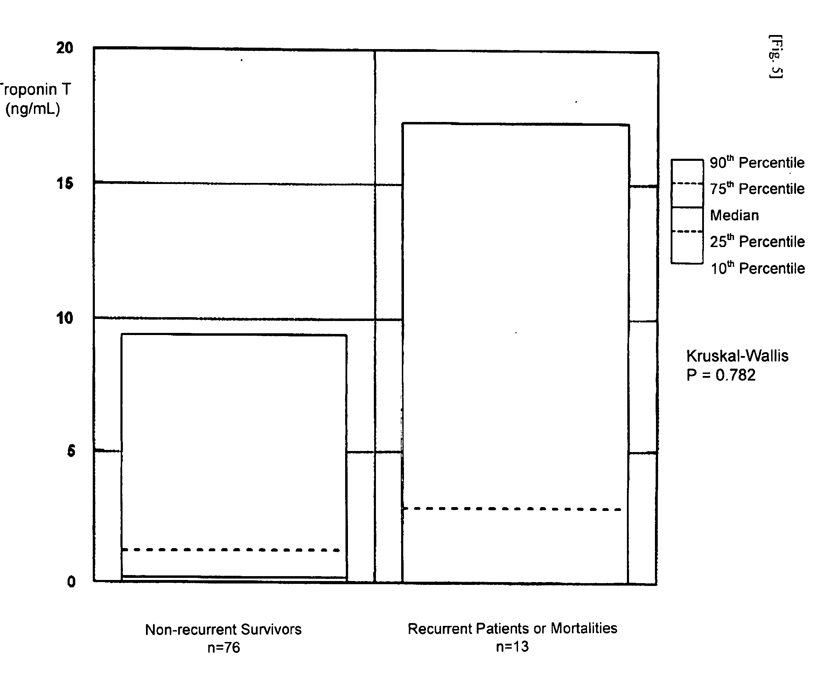

Test results showed that blood concentrations of soluble LOX-1 showed statistical significance of P<0.003 in the recurrent group and the non-recurrent group (Fig. 2). On the other hand, other markers and examination method, like blood high-sensitivity CRP levels (0.348), blood troponin T concentrations (0.782), and left ventricular ejection fraction (LVEF) (0.782), did not show any significance (Fig.3 through 5). In this way, unlike other markers and examination methods, only soluble LOX-1 could showed the high correlative relationship with acute coronary syndrome (including patients who died as a result).

### Working Example 3

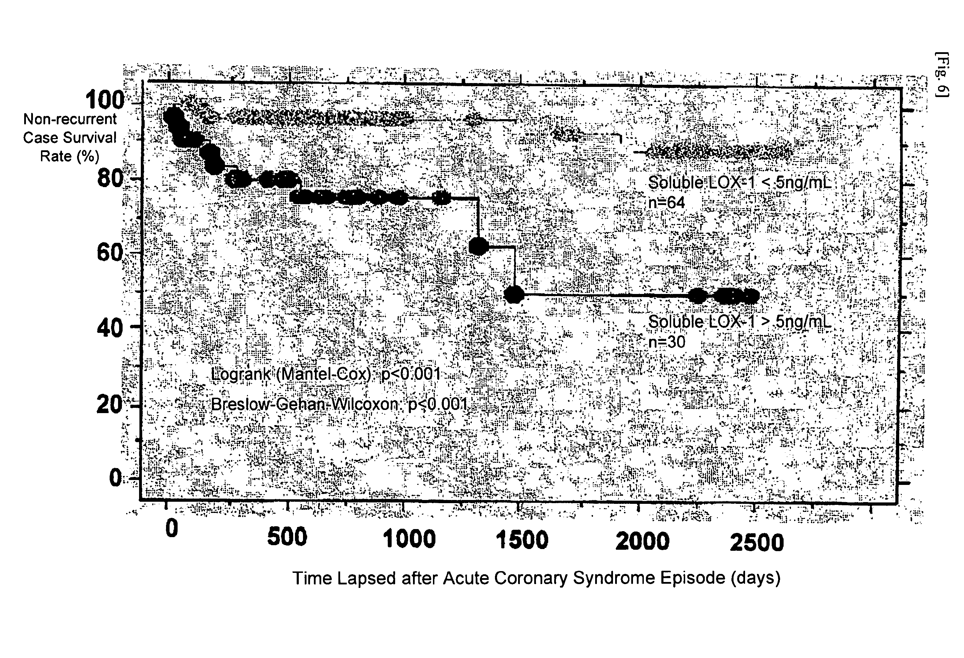

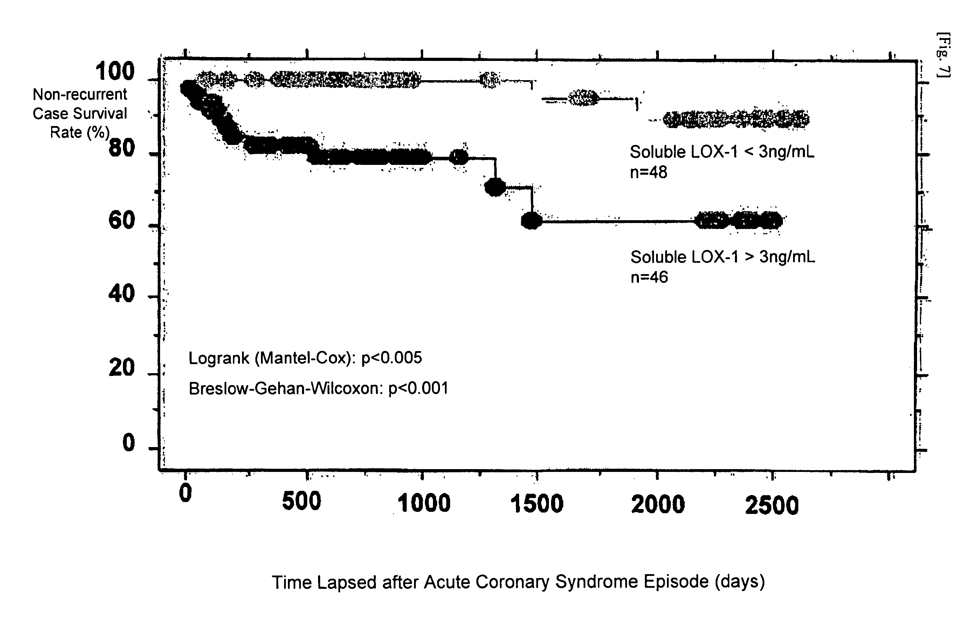

The Kaplan-Meir curve (Shinban Igaku heno toukeigaku, Asakura Syoten, 1993) for soluble LOX-1 was found based on statistical processing that took the time until recurrence or death into consideration. The cutoff was set at 3 ng/mL or 5 ng /mL .Whether the cutoff was 3 ng/mL or 5 ng/mL, the group of positive patients had recurrences of early-stage acute coronary syndrome or recurrences resulting in death at a higher frequency than the group of patients that tested negative (Fig. 6, Fig. 7).
These results show that soluble LOX-1 is not only useful as a primary diagnostic marker, but also as a marker for predicting a recurrence of acute coronary syndrome (prognostic prediction).

### [Industrial Applicability]

This invention makes it possible to accurately identify and intensively manage patients with high risk of recurrence of acute coronary syndrome, the numbers of which patients are expected to increase in the future.

## Claims

1. A method of identifying test subjects at high risk of recurrence of acute coronary syndrome, which includes a process to measure the concentration of soluble LOX-1 in a test specimen taken from the test subject, wherein the concentration is taken as a marker for recurrence of acute coronary syndrome.

2. The method disclosed in Claim 1, in which condition wherein the aforementioned concentration is greater than a reference value is taken that the aforementioned test subject is at high risk for recurrence of acute coronary syndrome.

3. The method disclosed in any of Claims 1 or 2, in which the aforementioned measurement is performed using an antibody or antibody fragment that specifically binds to human soluble LOX-1.

4. The method disclosed in Claim 3, in which the aforementioned measurement is performed by means of ELISA.

5. A kit for the implementation of the method in any of Claims 1 through 4.

6. The use of soluble LOX-1 as a marker for prognosis of the recurrence of acute coronary syndrome.


SEQUENCE LISTING

<110>  Shionogi & Co., Ltd.

<120>  Method of prognostic expectation for acute coronary syndromes

<130>  667216

<150>  JP 2005-370030
<151>  2005-12-22

<160>  3

<170>  PatentIn version 3.1


<210>  1
<211>  273
<212>  PRT
<213>  Homo sapiens

<400>  1

Met Thr Phe Asp Asp Leu Lys Ile Gln Thr Val Lys Asp Gln Pro Asp
1               5                   10                  15


Glu Lys Ser Asn Gly Lys Lys Ala Lys Gly Leu Gln Phe Leu Tyr Ser
            20                  25                  30


Pro Trp Trp Cys Leu Ala Ala Ala Thr Leu Gly Val Leu Cys Leu Gly
        35                  40                  45


Leu Val Val Thr Ile Met Val Leu Gly Met Gln Leu Ser Gln Val Ser
    50                  55                  60


Asp Leu Leu Thr Gln Glu Gln Ala Asn Leu Thr His Gln Lys Lys Lys
65                  70                  75                  80


Leu Glu Gly Gln Ile Ser Ala Arg Gln Gln Ala Glu Glu Ala Ser Gln
                85                  90                  95


Glu Ser Glu Asn Glu Leu Lys Glu Met Ile Glu Thr Leu Ala Arg Lys
            100                 105                 110


Leu Asn Glu Lys Ser Lys Glu Gln Met Glu Leu His His Gln Asn Leu
        115                 120                 125


Asn Leu Gln Glu Thr Leu Lys Arg Val Ala Asn Cys Ser Ala Pro Cys
    130                 135                 140


Pro Gln Asp Trp Ile Trp His Gly Glu Asn Cys Tyr Leu Phe Ser Ser
145                 150                 155                 160

Gly Ser Phe Asn Trp Glu Lys Ser Gln Glu Lys Cys Leu Ser Leu Asp
                165                 170                 175

Ala Lys Leu Leu Lys Ile Asn Ser Thr Ala Asp Leu Asp Phe Ile Gln
            180                 185                 190

Gln Ala Ile Ser Tyr Ser Ser Phe Pro Phe Trp Met Gly Leu Ser Arg
        195                 200                 205

Arg Asn Pro Ser Tyr Pro Trp Leu Trp Glu Asp Gly Ser Pro Leu Met
    210                 215                 220

Pro His Leu Phe Arg Val Arg Gly Ala Val Ser Gln Thr Tyr Pro Ser
225                 230                 235                 240

Gly Thr Cys Ala Tyr Ile Gln Arg Gly Ala Val Tyr Ala Glu Asn Cys
                245                 250                 255

Ile Leu Ala Ala Phe Ser Ile Cys Gln Lys Lys Ala Asn Leu Arg Ala
            260                 265                 270

Gln

<210>  2
<211>  186
<212>  PRT
<213>  Homo sapiens

<400>  2

Arg Gln Gln Ala Glu Glu Ala Ser Gln Glu Ser Glu Asn Glu Leu Lys
1               5                   10                  15

Glu Met Ile Glu Thr Leu Ala Arg Lys Leu Asn Glu Lys Ser Lys Glu
            20                  25                  30

Gln Met Glu Leu His His Gln Asn Leu Asn Leu Gln Glu Thr Leu Lys
        35                  40                  45

Arg Val Ala Asn Cys Ser Ala Pro Cys Pro Gln Asp Trp Ile Trp His
    50                  55                  60

Gly Glu Asn Cys Tyr Leu Phe Ser Ser Gly Ser Phe Asn Trp Glu Lys
65                  70                  75                  80

Ser Gln Glu Lys Cys Leu Ser Leu Asp Ala Lys Leu Leu Lys Ile Asn
                85                  90                  95

Ser Thr Ala Asp Leu Asp Phe Ile Gln Gln Ala Ile Ser Tyr Ser Ser
            100                 105                 110

Phe Pro Phe Trp Met Gly Leu Ser Arg Arg Asn Pro Ser Tyr Pro Trp
        115                 120                 125

Leu Trp Glu Asp Gly Ser Pro Leu Met Pro His Leu Phe Arg Val Arg
    130                 135                 140

Gly Ala Val Ser Gln Thr Tyr Pro Ser Gly Thr Cys Ala Tyr Ile Gln
145                 150                 155                 160

Arg Gly Ala Val Tyr Ala Glu Asn Cys Ile Leu Ala Ala Phe Ser Ile
                165                 170                 175

Cys Gln Lys Lys Ala Asn Leu Arg Ala Gln
            180                 185

<210>  3
<211>  182
<212>  PRT
<213>  Homo sapiens

<400>  3

Glu Glu Ala Ser Gln Glu Ser Glu Asn Glu Leu Lys Glu Met Ile Glu
1               5                   10                  15

Thr Leu Ala Arg Lys Leu Asn Glu Lys Ser Lys Glu Gln Met Glu Leu
            20                  25                  30

His His Gln Asn Leu Asn Leu Gln Glu Thr Leu Lys Arg Val Ala Asn
        35                  40                  45

Cys Ser Ala Pro Cys Pro Gln Asp Trp Ile Trp His Gly Glu Asn Cys
    50                  55                  60

Tyr Leu Phe Ser Ser Gly Ser Phe Asn Trp Glu Lys Ser Gln Glu Lys
65                  70                  75                  80

Cys Leu Ser Leu Asp Ala Lys Leu Leu Lys Ile Asn Ser Thr Ala Asp
                85                  90                  95

Leu Asp Phe Ile Gln Gln Ala Ile Ser Tyr Ser Ser Phe Pro Phe Trp
            100                 105                 110

Met Gly Leu Ser Arg Arg Asn Pro Ser Tyr Pro Trp Leu Trp Glu Asp
        115                 120                 125

Gly Ser Pro Leu Met Pro His Leu Phe Arg Val Arg Gly Ala Val Ser
    130                 135                 140

Gln Thr Tyr Pro Ser Gly Thr Cys Ala Tyr Ile Gln Arg Gly Ala Val
145                 150                 155                 160

Tyr Ala Glu Asn Cys Ile Leu Ala Ala Phe Ser Ile Cys Gln Lys Lys
                165                 170                 175

Ala Asn Leu Arg Ala Gln
            180